# EUROPÄISCHE PATENTANMELDUNG

(11) **EP 1 006 189 A2**
(43) Veröffentlichungstag der Anmeldung: **07.06.2000**
(21) Anmeldenummer: 99123738.9
(22) Anmeldetag: 30.11.1999
(51) Int. Cl.: C12N 15/52, C12N 15/54, C12N 15/60, C12N 15/77, C12P 13/02, C12N 1/21, C12R 1/15

(54) **Verfahren zur fermentativen Herstellung von D-Pantothensäure unter Verwendung coryneformer Bakterien**

(30) Priorität: 01.12.1998 DE 19855312
(71) Anmelder: Degussa-Hüls Aktiengesellschaft, 60287 Frankfurt am Main (DE); FORSCHUNGSZENTRUM JÜLICH GMBH, 52425 Jülich (DE)
(72) Erfinder: Eggeling, Lothar, Dr., 52428 Jülich (DE); Thierbach, Georg, Dr., 33613 Bielefeld (DE); Sahm, Herrmann, Prof.Dr., 52428 Jülich (DE)

(57) **Zusammenfassung**

Die Erfindung betrifft in Mikroorganismen der Gattung Corynebacterium replizierbare, gegebenfalls rekombinante DNA mit der Herkunft Corynebacterium, die zumindest eine der folgenden Nucleotidsequenzen enthält, ausgewählt aus der Gruppe:
a) codierend für das panB-Gen (Ketopantoathydroxymethyltranferase), dargestellt in der SEQ-ID-No.1,
b) codierend für das panC-Gen (Pantothenatsynthetase), dargestellt in der SEQ-ID-No.1, insbesondere das panBC-Operon und gegebenenfalls
c) codierend für das ilvD-Gen (Dihydroxysäuredehydratase), dargestellt durch die SEQ-ID-No.4,
und ein Verfahren zur fermentativen Herstellung von D-Pantothensäure unter Verwendung von Mikroorganismen der Gattung Corynebacterium, in denen die genannten Gene verstärkt werden.

## Beschreibung

### Stand der Technik

Die Pantothensäure stellt ein kommerziell bedeutendes Vitamin dar, das in der Kosmetik, der Medizin, der Humanernährung und in der Tierernährung Anwendung findet.

Pantothensäure kann durch chemische Synthese oder biotechnologisch durch Fermentation geeigneter Mikroorganismen in geeigneten Nährlösungen hergestellt werden. Der Vorteil der biotechnologischen Herstellung durch Mikroorganismen liegt in der Bildung der gewünschten stereo-isomeren D-Form der Pantothensäure.

Verschiedene Arten von Bakterien, wie z. B. Escherichia coli, Corynebacterium erythrogenes, Brevibacterium ammoniagenes und auch Hefen, wie z. B. Debaromyces castellii können, wie in EP-A 0 493 060 gezeigt, in einer Nährlösung, die Glucose, DL-Pantoinsäure und β-Alanin enthält, D-Pantothensäure produzieren. EP-A 0 493 060 zeigt weiterhin, daß bei Escherichia coli durch Amplifikation von Pantothensäure-Biosynthesegenen mittels der Plasmide pFV3 und pFV5 die Bildung von D-Pantothensäure verbessert wird.

EP-A 0 590 857 betrifft Stämme von Escherichia coli, die Resistenzen gegen verschiedene Antimetabolite, wie z. B. Salizylsäure, α-Ketobuttersäure, β-Hydroxyasparaginsäure etc. tragen und in einer Nährlösung, die Glucose und β-Alanin enthält, D-Pantoinsäure und D-Pantothensäure produzieren. In EP- 0 590 857 wird weiterhin beschrieben, daß durch Amplifikation von nicht näher definierten Pantothensäure-Biosynthesegenen aus E.coli, die auf dem Plasmid pFV31 enthalten sind, die Produktion von D-Pantoinsäure und D-Pantothensäure in E.coli verbessert werden kann.

In WO 97/10340 wird darüber hinaus gezeigt, daß in Pantothensäure bildenden Mutanten von Escherichia coli durch Erhöhung der Aktivität des Enzyms Acetohydroxysäure-Synthase II, einem Enzym der Valin Biosynthese, die Pantothensäure-Produktion weiter gesteigert werden kann.

### Aufgabe der Erfindung

Die Erfinder haben sich zur Aufgabe gestellt neue Grundlagen für verbesserte Verfahren zur fermentativen Herstellung von D-Pantothensäure mit Hilfe coryneformer Bakterien bereitzustellen.

### Beschreibung der Erfindung

Das Vitamin Pantothensäure stellt ein kommerziell bedeutendes Produkt dar, das in der Kosmetik, der Medizin, der Humanernährung und in der Tierernährung Anwendung findet. Es besteht daher ein allgemeines Interesse daran verbesserte Verfahren zur Herstellung von Pantothensäure bereitzustellen.
Wenn im folgenden Text D-Pantothensäure oder Pantothensäure oder Pantothenat erwähnt werden, sind damit nicht nur die freie Säure, sondern auch die Salze der D-Pantothensäure wie z.B. das Calcium-, Natrium-, Ammonium- oder Kaliumsalz gemeint.
Gegenstand der Erfindung sind in Mikroorganismen der Gattung Corynebacterium replizierbare, gegebenfalls rekombinante DNA mit der Herkunft Corynebacterium, die zumindest eine der folgenden Nucleotidsequenzen enthält, ausgewählt aus der Gruppe:
a) codierend für das panB-Gen (Ketopantoathydroxymethyltranferase), dargestellt in der SEQ-ID-No.1,
b) codierend für das panC-Gen (Pantothenatsynthetase), dargestellt in der SEQ-ID-No.1, insbesondere das panBC-Operon und gegebenenfalls
c) codierend für das ilvD-Gen (Dihydroxysäuredehydratase), dargestellt durch die SEQ-ID-No.4.

Gegenstand der Erfindung sind ebenso replizierbare DNA gemäß dem genannten Anspruch 1
mit:
(i) den Nucleotidsequenzen, gezeigt in SEQ-ID-No.1, SEQ-ID-No.4,
(ii) mindestens einer dieser Sequenzen, die den jeweiligen Sequenzen (i) innerhalb des Bereichs der Degeneration des genetischen Codes entsprechen oder
(iii) mindestens einer dieser Sequenzen, die mit den zu jeweiligen Sequenzen (i) oder (ii) komplementären Sequenzen hybridisieren und gegebenenfalls
(iiii) funktionsneutrale Sinnmutationen in (i).

Ebenso werden beansprucht coryneforme Mikroorganismen, insbesondere der Gattung Corynebacterium, transformiert durch die Einführung einer oder mehrer replizierbarer DNA-Stücke.
Gegenstand der Erfindung ist auch ein Verfahren zur Herstellung von D-Pantothensäure unter Verwendung, insbesondere diese Säure bereits produzierender coryneformer Bakterien, in denen die Gene panB und panC einzeln oder kombiniert miteinander gegebenenfalls kombiniert mit einer Defektmutation im ilvA-Gen oder einer Verstärkung der Gene ilvBN, ilvC oder ilvD verstärkt, insbesondere überexprimiert werden.

Der Begriff 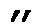 Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man z. B. die Kopienzahl des(der) Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können Pantothensäure aus Glucose, Saccharose, Lactose, Fructose, Maltose, Melasse, Stärke, Cellulose oder aus Glycerin und Ethanol herstellen, insbesondere aus Glucose oder Saccharose. Es handelt sich um coryneforme Bakterien z. B. der Gattungen Corynebacterium oder Arthrobacter. Bei der Gattung Corynebacterium ist insbesondere die Art Corynebacterium glutamicum zu nennen, die in der Fachwelt für ihre Fähigkeit bekannt, ist Aminosäuren zu bilden. Zu dieser Art gehören Wildtypstämme wie z. B. Corynebacterium glutamicum ATCC13032, Brevibacterium flavum ATCC14067, Corynebacterium melassecola ATCC17965 und davon abgeleitete Stämme.

Die Erfinder fanden heraus, dass nach Verstärkung, insbesondere Überexpression, der neu isolierten D-Pantothenatbiosynthesegene panB und panC einzeln oder gemeinsam (panBC-Operon) aus Corynebacterium glutamicum, die für die Enzyme Ketopantoathydroxymethyltransferase und Pantothenatesynthetase kodieren, in verbesserter Weise D-Pantothenat produziert wird.

Die Erfinder haben weiter festgestellt, daß eine verstärkte Expression des neuen Valinbiosynthesegens ilvD aus Corynebacterium glutamicum, welches für das Enzym Dihydroxysäuredehydratase kodiert, zur erhohten D-Pantothenatbildung beiträgt. Erfindungsgemäss bewirken neben diesem Gen auch die verstärkte Expression der ilvBN-Gene, die für das Enzym Acetohydroxysäuresynthase kodieren, und des ilvC-Gens, das für das Enzym Isomeroreduktase kodiert, in Corynebacterium glutamicum eine erhöhte D-Pantothenatbildung.

Zur Erzielung einer Verstärkung (Überexpression) erhöht man z. B. die Kopienzahl der entsprechenden Gene oder mutiert die Promotor- und Regulationsregion, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich die Expression im Verlaufe der fermentativen D-Pantothenatbildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmidvektoren mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Jensen und Hammer (Biotechnology and Bioengineering 58, 191-195 (1998)) oder im Handbuch 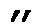 Manual of Methods for General Bacteriology der American Society for Bacteriology (Washington D.C., USA, 1981) und in bekannten Lehrbüchern der Genetik und Molekularbiologie.

Zur Isolierung der Gene panB und panC aus C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 ( Raleigh et al., 1988, Nucleic Acids Research 16:1563-1575) angelegt wurde. Zur Herstellung einer Genbank von C. glutamicum in E. coli können auch Plasmide wie pBR322 (Bolivar, Life Sciences, 25, 807-818 (1979)) oder pUC19 (Norrander et al., 1983, Gene, 26: 101-106) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde.

Die Genbank wird anschließend in einen Indikatorstamm durch Transformation (Hanahan, Journal of Molecular Biology 166, 557-580, 1983) oder Elektroporation (Tauch et.al., 1994, FEMS Microbiological Letters, 123:343-347) eingebaut. Der Indikatorstamm zeichnet sich dadurch aus, dass er eine Mutation in dem interessierenden Gen besitzt, die einen detektierbaren Phänotyp z.B. eine Auxotrophie hervorruft. Die Indikatorstämme bzw. Mutanten sind aus publizierten Quellen oder Stammsammlungen erhältlich oder werden gegebenfalls selbst hergestellt. Im Rahmen der vorliegenden Erfindung ist die E. coli Mutante DV39 (Vallari und Rock, Journal of Bacteriology 1985, 164:136-142), die eine Mutation im panC-Gen trägt, von besonderem Interesse. Ein anderes Beispiel für eine Pantothensäure-bedürftige E. coli Mutante ist der Stamm SJ2, der eine Mutation im panB-Gen trägt und vom Genetic Stock Center der Yale University (New Haven, Connecticut, USA) bezogen werden kann. Ein weiteres Beispiel ist die im Rahmen der vorliegenden Erfindung isolierte C. glutamicum Mutante R127/7, die in dem für die Dihydroxysäuredehydratase kodierendem ilvD-Gen defekt ist. Nach Transformation des Indikatorstammes wie z.B. der panB-Mutante SJ2 mit einem rekombinanten Plasmid, welches das interessierende Gen wie z.B. das panB-Gen trägt, und Expression des betreffenden Gens, wird der Indikatorstamm bezüglich der entsprechenden Eigenschaft wie z.B. der Pantothensäure-Bedürftigkeit prototroph.

Das dergestalt isolierte Gen bzw. DNA-Fragment kann durch Bestimmung der Sequenz, wie z.B. bei Sanger et al. (Proceedings of the National of Sciences of the United States of America USA, 74:5463-5467, 1977) beschrieben, charakterisiert werden.

Auf diese Weise wurde die neue für die Gene panB und panC kodierende DNA-Sequenz von C. glutamicum erhalten, die als SEQ ID NO 1 Bestandteil der vorliegenden Erfindung ist. Weiterhin wurden aus der vorliegenden DNA-Sequenz mit den oben beschriebenen Methoden die Aminosäuresequenzen der entsprechenden Enzyme abgeleitet. In SEQ ID NO 2 ist die sich ergebende Aminosäuresequenz des panB-Genproduktes nämlich der Ketopantoathydroxymethyltransferase und in SEQ ID NO 3 die sich ergebende Aminosäuresequenz des panC-Genproduktes nämlich der Pantothenatsynthetase dargestellt. Weiterhin wurde auf diese Weise die neue für das ilvD-Gen kodierende DNA-Sequenz von C. glutamicum erhalten, die als SEQ ID NO 4 Bestandteil der vorliegenden Erfindung ist. In SEQ ID NO 5 ist die sich ergebende Aminosäuresequenz des ilvD-Genproduktes nämlich der Dihydroxysäuredehydratase dargestellt.

Kodierende DNA-Sequenzen, die sich aus SEQ ID NO 1 und/oder SEQ ID NO 4 durch einen degenerierten genetischen Code ergeben, sind ebenfalls Bestandteil der Erfindung. In gleicher Weise sind DNA-Sequenzen, die mit SEQ ID NO 1 und/oder SEQ ID NO 4 hybridisieren Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als 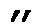 Sinnmutationen" (sense mutations) bekannt, die zu keiner grundsätzlichen Veränderung der Aktivität des Proteins führen d.h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2, SEQ ID NO 3 und/oder SEQ ID NO 5 ergeben sind ebenfalls Bestandteil der Erfindung.

Das dergestalt charakterisierte Gen kann anschließend einzeln oder in Kombination mit anderen in einem geeigneten Mikroorganismus zur Expression gebracht werden. Eine bekannte Methode Gene zu exprimieren bzw. überzuexprimieren besteht darin diese mit Hilfe von Plasmidvektoren zu amplifizieren, die überdies mit Expressionssignalen ausgestattet sein können. Als Plasmidvektoren kommen solche in Frage, die in den entsprechenden Mikroorganismen replizieren können. Für Corynebacterium glutamicum kommen z.B. die Vektoren pEKEx1 (Eikmanns et al., Gene 102:93-98 (1991)) oder pZ8-1 (Europäische Patentschrift 0 375 889) oder pEKEx2 (Eikmanns et al. Microbiology 140: 1817-1828 (1994) oder pECM2 (Jäger et al. Journal of Bacteriology 174(16): 5462-5465 (1992)) in Frage. Beispiele für derartige Plasmide sind pEKEx2panBC und pECM3ilvBNCD, die in den Stämmen DH5αmcr/pEKEx2panBC und DH5αmcr/pECM3ilvBNCD enthalten sind. Plasmid pEKEx2panBC ist ein E. coli/C. glutamicum Pendelvektor der die Gene panB und panC trägt. Plasmid pECM3ilvBNCD ist ein E. coli/C. glutamicum Pendelvektor der neben dem ilvD-Gen weiterhin die Gene ilvBN und ilvC trägt.

Die Erfinder haben weiterhin gefunden, dass sich die Verstärkung der Gene panB und panC einzeln, gemeinsam oder in Kombination mit den Genen ilvBN, ilvC und ilvD in solchen Mikroorganismen vorteilhaft auswirkt, die eine reduzierte Synthese der Aminosäuren Threonin und Isoleucin aufweisen. Diese reduzierte Synthese kann durch Abschwächung oder Ausschaltung der entsprechenden Biosyntheseenzyme bzw. ihrer Aktivitäten erreicht werden. Hierfür kommen zum Beispiel die Enzyme Homoserindehydrogenase, Homoserinkinase, Threoninsynthase oder auch Threonindehydratase in Frage. Eine Möglichkeit Enzyme und deren Aktivitäten abzuschwächen oder auszuschalten sind Mutageneseverfahren.

Hierzu gehören ungerichtete Verfahren, die chemische Reagenzien wie z.B. N-methyl-N-nitro-N-nitrosoguanidin oder auch UV-Bestrahlung zur Mutagenese benutzen, mit anschließender Suche der gewünschten Mikroorganismen auf Bedürftigkeit für L-Threonin oder L-Isoleucin. Verfahren zur Mutationsauslösung und Mutantensuche sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden.

Weiterhin gehören hierzu gerichtete rekombinante DNA-Techniken. Mit Hilfe dieser Methoden kann zum Beispiel das für die Threonindehydratase kodierende ilvA-Gen im Chromosom deletiert wird. Geeignete Methoden dazu sind bei Schäfer et al. (Gene (1994) 145: 69-73) oder auch Link et al. (Journal of Bacteriology (1998) 179: 6228-6237) beschrieben. Auch können nur Teile des Gens deletiert werden, oder auch mutierte Fragmente des Threonindehydratasegens ausgetauscht werden. Durch Deletion oder Austausch wird so ein Verlust oder eine Reduktion der Threonindehydrataseaktivität erreicht (Möckel et al., (1994) Molecular Microbiology 13: 833-842; Morbach et al., (1996) Applied Microbiology and Biotechnology 45: 612-620). Ein Beispiel für eine derartige Mutante ist der C. glutamicum Stamm ATCC13032ΔilvA, der eine Deletion im ilvA-Gen trägt.

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Pantothensäure-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies zur zusätzlichen Steigerung der Pantothensäure-Produktion Vorstufen der Pantothensäure wie z. B. Aspartat, β-Alanin; Ketolsovalerat, Ketopantoat, Pantoat und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 50°C und vorzugsweise bei 25°C bis 45°C. Die Kultur wird solange fortgesetzt bis sich ein Maximum an Pantothensäure gebildet hat. Dieses Ziel wird normalerweise innerhalb von 10 Stunden bis 160 Stunden erreicht.

Die Konzentration an gebildeter Pantothensäure kann mit bekannten Verfahren (Velisek; Chromatographic Science 60, 515-560 (1992)) bestimmt werden. Zur mikrobiologischen Bestimmung von Pantothensäure wird gebräuchlicherweise der Stamm Lactobacillus plantarum ATCC8014 eingesetzt (U.S. Pharmacopeia 1980; AOAC International 1980). Darüberhinaus werden auch andere Testorganismen, wie z.B. Pediococcus acidilactici NCIB6990 zur mikrobiologischen Bestimmung von Pantothenatkonzentrationen eingesetzt (Sollberg and Hegna; Methods in Enzymology 62, 201-204 (1979)).

Folgende Mikroorganismen wurden bei der Deutschen Sammlung für Mikrorganismen und Zellkulturen (DSMZ, Braunschweig, Deutschland) gemäß Budapester Vertrag hinterlegt:
- Escherichia coli K12 Stamm DH5αmcr/pEKEx2panBC als DSM12456
- Escherichia coli K12 Stamm DH5αmcr/pECM3ilvBNCD als DSM12457
- Corynebacterium glutamicum ATCC13032ΔilvA als DSM12455

### Beispiele

Die vorliegende Erfindung wird im folgenden anhand von Ausführungsbeispielen näher erläutert.

### Beispiel 1

### Klonierung, Sequenzierung und Expression der Gene der Pantothenatbiosynthese panB und panC aus C. glutamicum

### 1. Klonierung des panB- und des panC-Gens

Chromosomale DNA von C. glutamicum ATCC13032 wurde wie bei Schwarzer und Pühler (Bio/Technology 9 (1990) 84-87) beschrieben isoliert und mit der Restriktionsendonuklease Sau3A geschnitten. Nach geelektrophoretischer Auftrennung wurden DNA-Fragmente in einem Größenbereich von 3 bis 7 kb bzw. von 9 bis 20 kb extrahiert und nachfolgend in die singuläre BamHI Schnittstelle des Vektors pBR322 ligiert. Mit den Ligationsansätzen wurde der E. coli Stamm DH5αmcr (Grant et al., Proceedings of the National Academy of Sciences of the United States of America USA, 87 (1990) 4645-4649) transformiert (Hanahan, Journal of Molecular Biology 166 (1983) 557-580). Inserttragende Kolonien wurden anhand ihrer Tetracyclinsensitivität nach Überimpfen auf 10 µg/ml Tetracyclin enthaltende LB-Agarplatten identifiziert. Durch Plasmidpräparationen (Sambrook et al., Molecular cloning. A laboratory manual (1989) Cold Spring Harbour Laboratory Press) von vereinigten Klonen wurden 8 Gruppen, welche je 400 Plasmide mit einer Insertgröße von 9 bis 20 kb und 9 Gruppen, welche je 500 Plasmide mit einer Insertgröße von 3 bis 7 kb enthielten isoliert. Die E. coli panB Mutante SJ2 (Cronan et al. 1982, Journal of Bacteriology 149: 916-922) wurde mit dieser Genbank mittels Elektroporation (Wehrmann et al. 1994, Microbiology 140: 3349-3356) transformiert. Die Transformationsansätze wurden direkt auf CGXII-Medium mit 15 g/l Agar (Keilhauer et al., Journal of Bacteriology (1993) 175: 5595-5603) ausplattiert. Von Klonen, welche in der Lage waren ohne Pantothenatsupplementation zu wachsen, wurde Plasmid-DNA isoliert (Sambrook et al., Molecular cloning. A laboratory manual (1989) Cold Spring Harbour Laboratory Press). Bei 8 Plasmiden konnte durch Retransformation die Fähigkeit, den panB-Defekt der E. coli Mutante SJ2 heterolog zu komplementieren, bestätigt werden.

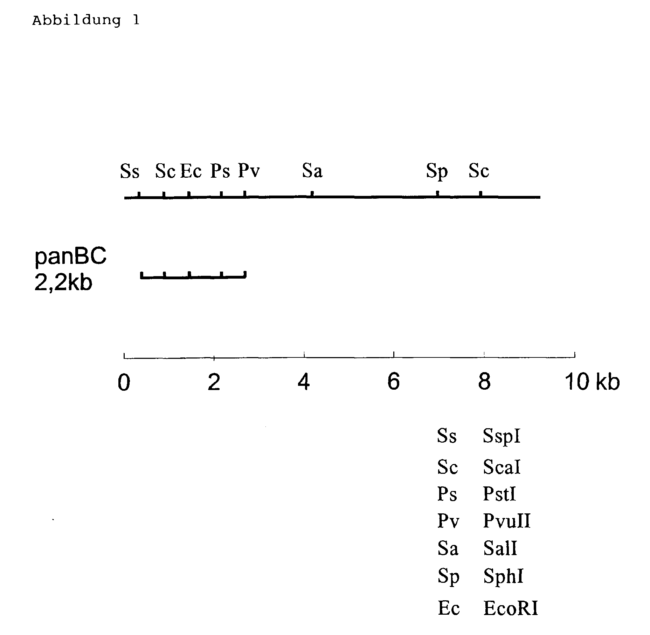

Mit diesen 8 Plasmiden wurde eine Restriktionskartierung durchgeführt. Einer der untersuchten Plasmidvektoren, im Folgendem pUR1 genannt enthielt ein Insert von 9,3 kb Länge (Abbildung 1). Die Transformation der E. coli panC Mutante DV39 (Vallari und Rock 1985, Journal of Bacteriology 164: 136-142) ergab, daß der Vektor pUR1 ebenfalls in der Lage war den panC Defekt dieser Mutante zu komplementieren.

### 2. Sequenzierung des panB- und des panC-Gens

Ein 2,2 kb großes Fragment des Inserts (Abbildung 1) von pUR1 wurde nach der Dideoxy-Kettenabbruchmethode von Sanger et al. sequenziert (Proceedings of the National Academy of Sciences of the United States of America USA (1977) 74: 5463-5467). Hierzu wurden zunächst mittels Exonuklease III Subklone erzeugt, die mit Hilfe von Standard Primern (Universal und reverse primer der Firma Boehringer Mannheim, Deutschland) sequenziert wurden. Die gelelektrophoretische Analyse der Sequenzieransätze erfolgte mit dem automatischem Laser-Fluoreszenz Sequenziergerät (A.L.F.) von Amersham Pharmacia Biotech (Uppsala, Schweden). Die erhaltene Nukleotidsequenz wurde mit dem Programmpaket HUSAR (Release 4.0, EMBL, Cambridge, GB) analysiert. Die Nukleotidsequenz ist als SEQ ID NO 1 wiedergegeben. Die Analyse ergab die Identizierung von zwei offenen Leserastern. Ein offenes Leseraster von 813 bp Länge, das als panB-Gen identifiziert wurde, kodiert für ein Polypeptid von 271 Aminosäuren und ist als SEQ ID NO 2 wiedergegeben. Das zweite offene Leseraster, das als panC-Gen identifiziert wurde, umfaßt 837 Basenpaare. Es kodiert für ein Polypeptid von 279 Aminosäuren, das als SEQ ID NO 3 wiedergegeben ist.

### 3. Expression des panB- und des panC-Gens

Die Gene panB und panC wurden in den C. glutamicum Expressionsvektor pEKEx2 kloniert (Eikmanns et al. 1994, Microbiology 140: 1817-1828 (1994)), in welchem die beiden Gene unter der Kontrolle des starken, durch IPTG induzierbaren tac-Promotors vorliegen. Die Klonierung wurde in zwei Schritten durchgeführt. Zunächst wurde mittels PCR der Anfang des panB Gens amplifiziert. Hierzu wurde mit Hilfe eines emtsprechenden Primers 19 bp vor dem Startcodon von panB eine SalI-Schnittstelle eingefügt (Primer 1: 5'GATCGTCGACCATCACATCTATACTCATGCCC 3'). Der zweite Primer wurde so gewählt, daß die panB interne EcoRI Schnittstelle im amplifizierten Fragment enthalten war (Primer 2: 5'ACCCG ATGTGGCCGACAACC 3'). Die PCR wurde mit einer Annealingtemperatur von 62°C und dem Plasmid pUR1 als Matrize nach Sambrook et al. (Molecular cloning. A laboratory manual, Cold Spring Harbour Laboratory Press (1989)) durchgeführt. Das resultierende 468 bp große PCR-Produkt wurde mit den Restriktionsendonukleasen SalI und EcoRI geschnitten und in den ebenso behandelten Vektor pEKEx2 ligiert. Mit dem Ligationsansatz wurde der E. coli Stamm DH5αmcr transformiert. Aus einer Transformante vom Typ DH5αmcr/pEKEx2panB' wurde der Vektor pEKEx2panB' isoliert.

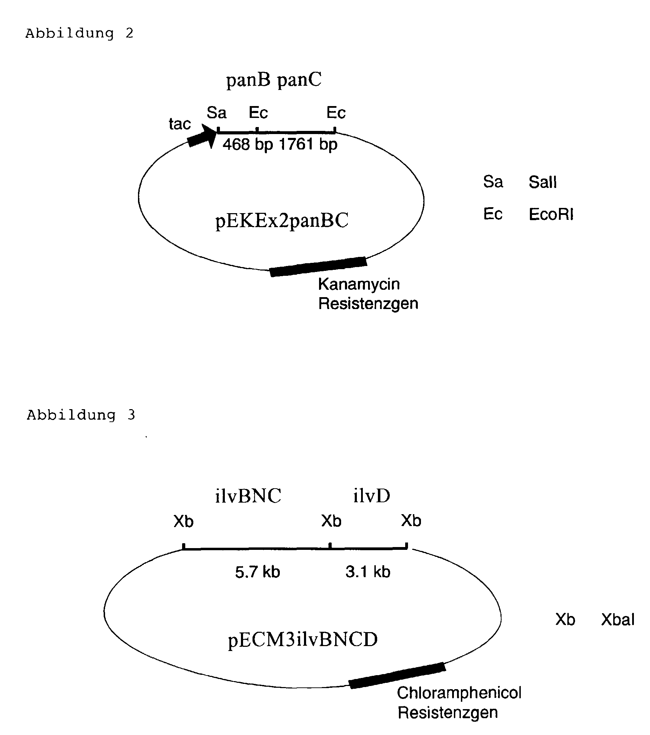

Aus dem Plasmid pUR1 wurde nun ein 1761 bp großes, die zweite Hälfte des panBC-Clusters enthaltendes, EcoRI Fragment mittels Restriktionsverdau ausgeschnitten. Dieses wurde in den schon das panB PCR-Produkt enthaltenden, zuvor mit EcoRI linearisierten pEKEx2panB' Vektor kloniert. Mit dem entsprechenden Ligationsansatz wurde der E. coli Stamm DH5αmcr transformiert. Aus einer Transformante vom Typ DH5αmcr/pEKEx2panBC wurde der Vektor pEKEx2panBC (Abbildung 2) isoliert, in dem das panBC-Gencluster unter der Kontrolle des tac-Promotors vorliegt.

### Beispiel 2

### Klonierung und Sequenzierung des für die Dihydroxysäuredehydratase kodierenden ilvD-Gens aus C. glutamicum

### 1. Isolierung einer ilvD Mutante von C. glutamicum

Der Stamm C. glutamicum R127 (Haynes 1989, FEMS Microbiology Letters 61: 329-334) wurde mit N-methyl-N-nitro-N-nitrosoguanidin mutagenisiert (Sambrook et al., Molecular cloning. A laboratory manual (1989) Cold Spring Harbour Laboratory Press). Dazu wurden 5 ml einer über Nacht angezogenen C. glutamicum Kultur mit 250 µl N-methyl-N-nitro-N-nitrosoguanidin (5 mg/ml Dimethylformamid) versetzt und 30 Minuten bei 30°C und 200 Upm inkubiert (Adelberg 1958, Journal of Bacteriology 76: 326). Die Zellen wurden anschließend zweimal mit steriler NaCl-Lösung (0,9 %) gewaschen. Durch Replikaplattierung auf Minimalmediumplatten CGXII mit 15 g/l Agar (Keilhauer et al Journal of Bacteriology 175: 5595-5603), wurden Mutanten isoliert, die nur bei Zugabe von L-Valin, L-Isoleucin und L-Leucin (je 0,1 g/l) wuchsen.

Die Enzymaktivität der Dihydroxysäuredehydratase wurde im Rohextrakt dieser Mutanten bestimmt. Dazu wurden die Klone in 60 ml LB-Medium kultiviert und in der exponentiellen Wachstumsphase abzentrifugiert. Das Zellpellet wurde einmal mit 0,05 M Kaliumphosphatpuffer gewaschen und im selben Puffer resuspensiert. Der Zellaufschluß erfolgte mittels 10 minütiger Ultraschallbehandlung (Branson-Sonifier W-250, Branson Sonic Power Co, Danbury, USA). Anschließend wurden die Zelltrümmer durch eine 30 minütige Zentrifugation bei 13000 rpm und 4 °C abgetrennt und der Überstand als Rohextrakt in den Enzymtest eingesetzt. Der Reaktionsansatz des Enzymtests enthielt 0,2 ml 0,25 M Tris/HCl, pH 8, 0,05 ml Rohextrakt, und 0,15 ml 65 mM alpha,β-Dihydroxy-β-methylvalerat. Die Testansätze wurden bei 30 °C inkubiert, nach 10, 20 und 30 Minuten 200 µl Proben genommen und deren Ketomethylvaleratkonzentration mittels HPLC-Analytik bestimmt (Hara et al. 1985, Analytica Chimica Acta 172: 167-173). Wie Tabelle 1 zeigt, weist der Stamm R127/7 keine Dihydroxysäuredehydrataseaktivität auf, wogegen die Isomeroreduktase und Acetohydroxysäuresynthase Aktivitäten als weitere Enzyme der Synthese der verzweigtkettigen Aminosäuren noch vorhanden sind.

**Tabelle 1**

| Spezifische Aktivitäten (µmol/min und mg Protein) verschiedener Enzyme in C. glutamicum Stämmen | | | |
|---|---|---|---|
| Stamm | Dihydroxysäure dehydratase | Isomero reduktase | Acetohydroxysäure synthase |
| R127 | 0,003 | 0,05 | 0,07 |
| R127/7 | 0,000 | 0,06 | 0,09 |

### 2. Klonierung des ilvD-Gens von C. glutamicum

Chromosomale DNA aus C. glutamicum R127 wurde wie bei Schwarzer und Pühler (Bio/Technology 9 (1990) 84-87) beschrieben isoliert. Diese wurde mit dem Restriktionsenzym Sau3A (Boehringer Mannheim) gespalten und durch Saccharose-Dichte-Gradienten-Zentrifugation (Sambrook et al., Molecular cloning. A laboratory manual (1989) Cold Spring. Harbour Laboratory Press) aufgetrennt. Die Fraktion mit dem Fragmentgrößenbereich von etwa 6-10 kb wurde zur Ligation mit dem Vektor pJC1 (Cremer et al., Molecular and General Genetics 220 (1990) 478-480) eingesetzt. Der Vektor pJC1 wurde hierzu mit BamHI linearisiert und dephosphoryliert. Fünf ng davon wurden mit 20 ng der genannten Fraktion der chromosomalen DNA ligiert und damit die Mutante R127/7 durch Elektroporation (Haynes und Britz, FEMS Microbiology Letters 61 (1989) 329-334) transformiert. Die Transformanten wurden auf die Fähigkeit getestet auf CGXII Agarplatten ohne Zugabe der verzweigtkettigen Aminosäuren wachsen zu können. Von über 5000 getesteten Transformanten wuchsen nach Replicaplattierung und zweitägiger Inkubation bei 30°C 8 Klone auf Minmalmediumplatten. Von diesen Klonen wurden Plasmidpräparationen, wie bei Schwarzer et al. (Bio/Technology (1990) 9: 84-87) beschrieben durchgeführt. Restriktionsanalysen der Plasmid-DNA ergaben, daß in allen 8 Klonen dasselbe Plasmid, im Folgendem pRV genannt, enthalten war. Das Plasmid trägt ein Insert von 4,3 kb und wurde durch Retransformation auf seine Fähigkeit die ilvD-Mutante R127/7 zu komplementieren getestet. Durch Subklonierung wurde der für die Komplementation der Mutante R127/7 verantwortliche Bereich auf ein 2,9 kb ScaI/XhoI-Fragment eingegrenzt.

### 3. Sequenzierung des ilvD-Gens

Die Nukleinsäuresequenz des 2,9 kb ScaI/XhoI-Fragments wurde nach der Dideoxy-Kettenabbruchmethode von Sanger et al. durchgeführt (Proceedings of the National of Sciences of the United States of America USA (1977) 74: 5463-5467). Dabei wurde der Auto-Read Sequencing kit verwendet (Amersham Pharmacia Biotech, Uppsala, Schweden). Die gelelektrophoretische Analyse erfolgte mit dem automatischem Laser-Fluoreszenz Sequenziergerät (A.L.F.) von Amersham Pharmacia Biotech (Uppsala, Schweden). Die erhaltene Nukleotidsequenz wurde mit dem Programmpaket HUSAR (Release 4.0, EMBL, Cambridge, GB) analysiert. Die Nukleotidsequenz ist als ID SEQ NO 4 wiedergegeben. Die Analyse ergab ein offenes Leseraster von 1836 Basenpaaren, das als ilvD-Gen identifiziert wurde und für ein Polypeptid von 612 Aminosäuren kodiert, das als SEQ ID NO 5 wiedergegeben ist.

### Beispiel 3

### Konstruktion einer ilvA Deletionsmutante von C. glutamicum

Der Einbau einer Deletion in das ilvA-Gen von Corynebacterium glutamicum ATCC13032 wurde mit dem bei Schäfer et al. (Gene 145: 69-73 (1994)) beschriebenen System zum Genaustausch durchgeführt. Zur Konstruktion des Inaktivierungsvektors pK19mobsacBΔilvA wurde zunächst aus dem auf einem EcoRI-Fragment im Vektor pBM21 (Möckel et al. 1994, Molecular Microbiology 13: 833-842) vorliegenden ilvA-Gen ein internes 241 bp BglII-Fragment entfernt. Hierzu wurde der Vektor mit BglII geschnitten und, nach Abtrennung des ilvA internen BglII-Fragmentes mittels Agarosegelelektrophorese, religiert. Anschließend wurde aus dem Vektor das unvollständige Gen als EcoRI-Fragment isoliert und in den mit EcoRI linearisierten Vektor pK19mobsacB (Schäfer 1994, Gene 145: 69-73) ligiert. Der erhaltene Inaktivierungsvektor pK19mobsacBΔilvA wurde durch Transformation in den E. coli Stamm S 17-1 eingebracht (Hanahan 1983, Journal of Molecular Biology 166: 557-580) und per Konjugation nach C. glutamicum ATCC13032 transferiert (Schäfer et al. 1990, Journal of Bacteriology 172: 1663-1666). Es wurden Kanamycin-resistente Klone von C. glutamicum erhalten, bei denen der Inaktivierungsvektor im Genom integriert vorlag. Um auf die Excision des Vektors zu selektionieren, wurden Kanamycin-resistente Klone auf Saccharose-haltigem LB-Medium ((Sambrook et al., Molecular cloning. A laboratory manual (1989) Cold Spring Harbour Laboratory Press)) mit 15 g/l Agar, 2% Glucose/ 10% Saccharose) ausplattiert und Kolonien erhalten, welche den Vektor durch ein zweites Rekombinationsereignis wieder verloren haben (Jäger et al. 1992, Journal of Bacteriology 174: 5462-5465). Durch Überimpfen auf Minimalmediumplatten (Medium CGXII mit 15 g/l Agar (Keilhauer et al., Journal of Bacteriology 175 (1993) 5595-5603)) mit und ohne 2 mM L-Isoleucin, bzw. mit und ohne 50 µg/ml Kanamycin wurden 36 Klone isoliert, welche durch die Excision des Vektors Kanamycin sensitiv und Isoleucin auxotroph waren und bei denen nun das unvollständige ilvA Gen (ΔilvA-Allel) im Genom vorlag. Einer dieser Klone wurde als Stamm ATCC13032ΔilvA bezeichnet und weiter verwendet.

### Beispiel 4

### Expression der Gene ilvBN, ilvC und ilvD in C. glutamicum

Die Gene der Acetohydroxysäuresynthase (ilvBN) und der Isomeroreduktase (ilvC) (Cordes et al. 1992, Gene 112: 113-116 und Keilhauer et al. 1993, Journal of Bacteriology 175: 5595-5603) und der Dihydroxysäuredehydratase (ilvD) (Beispiel 2) wurden zur Expression in den Vektor pECM3 kloniert. Der Vektor pECM3 ist ein Derivat von pECM2 (Jäger et al. 1992, Journal of Bacteriology 174: 5462-5465), das durch Deletion des ca. 1 kbp langen BamHI/BglII DNA-Fragmentes entstand, welches das Kanamycinresistenzgen trägt.

In dem Vektor pKK5 (Cordes et al. 1992, Gene 112: 113-116) lagen die Gene ilvBNC bereits im Vektor pJC1 (Cremer et al. 1990, Molecular and General Genetics 220: 478-480) kloniert vor. Aus diesem wurde ein 5,7 kb XbaI-ilvBNC-Fragment isoliert und zusammen mit einem, das ilvD-Gen enthaltende, 3,1 kb-XbaI Fragment des Vektors pRV in den mit XbaI linearisierten Vektor pECM3 eingebracht. Der Ligationsansatz wurde hierbei in den E. coli Stamm DH5αmcr transformiert. Aus einer Transformante vom Typ DH5αmcr/pECM3ilvBNCD wurde das Plasmid pECM3ilvBNCD (Abbildung 3) erhalten.

Mittels Elektroporation (Haynes 1989, FEMS Microbiology Letters 61: 329-334) und Selektion auf Chloramphenicolresistenz wurde das Plasmid pECM3ilvBNCD in den Stamm ATCC13032ΔilvA eingebracht und der Stamm ATCC13032ΔilvA/pECM3ilvBNCD erhalten. Weiterhin wurde mittels Elektroporation (Haynes 1989, FEMS Microbiology Letters 61: 329-334) und Selektion auf Kanamycinresistenz das Plasmid pEKEx2panBC in den Stamm ATCC13032 und in den Stamm ATCC13032ΔilvA eingebracht und die Stämme ATCC13032/pEKEx2panBC und ATCC13032ΔilvA/pEKEx2panBC erhalten. In den Stamm ATCC13032ΔilvA/pECM3ilvBNCD wurden mittels Elektroporation (Haynes 1989, FEMS Microbiology Letters 61: 329-334) und Selektion auf Kanamycin und Chloramphenicol die Plasmide pEKEx2panBC und pEKEX2 eingebracht. Auf diese Weise entstanden die Stämme ATCC13032ΔilvA/pECM3ilvBNCD pEKEX2 und ATCC13032ΔilvA/pECM3ilvBNCD pEKEx2panBC.

### Beispiel 5

### Konstruktion einer Pantothensäure bedürftigen panC-Mutante von C. glutamicum

Es wurde mit Hilfe des Inaktivierungsvektors pK18mob (Schäfer et al. 1994, Gene 145: 69-73) eine C. glutamicum R127 panC Mutante erzeugt.

Zur Konstruktion des panC-Inaktivierungsvektors wurde zunächst ein 168 bp großes, zentrales Fragment des panC-Gens (Nukleotid 265-432 des 837 bp umfassenden Gens) von C. glutamicum mittels der Polymersasekettenreaktion (PCR) amplifiziert. Als Matrize diente hier der Vektor pUR1 (s. Beispiel 6); als Primer wurden die beiden 20mere Primer 1 und Primer 2 eingesetzt: Primer 1 5' GTTCGCACCCGATGTGGAGG 3', Primer 2 5' ATGCACGATCAGGGCGCACC 3'. Die PCR wurde nach Sambrook et al. (Molecular cloning. A laboratory manual (1989) Cold Spring Harbour Laboratory Press) mit einer Annealingtemperatur von 55°C durchgeführt. Das erhaltene Fragment wurde nach Zwischenklonierung in die SmaI Schnittstelle des Vektors pUC18, als EcoRI/SalI Fragment gerichtet in den Inaktivierungsvektor pK18mob (Schäfer et al. 1994, Gene 145: 69-73) ligiert. Der so erhaltene Vektor pK18mob'panC' wurde zur Transformation des E. coli-Stammes S 17-1 benutzt und nachfolgend per Konjugation in C. glutamicum R127 eingebracht. Durch Selektion auf Kanamycinresistenz wurden so Klone von C. glutamicum R127 erhalten, bei denen der Integrationsvektor durch ein homologes Rekombinationsereignis ins panC-Gen integriert ist. Der so erhaltene Stamm R12YpanC::pK18mob'panC' ist zur D-Pantothenatbestimmung geeignet.

### Beispiel 6

### Quantitative Bestimmung von D-Pantothenat

Zur quantitativen Bestimmung von D-Pantothenat wurde die C. glutamicum panC Mutante R127panC::pK18mob'panC' konstruiert (siehe Beispiel 5), deren Wachstum direkt von der D-Pantothenat Konzentration des Mediums abhängig ist. Dieser Stamm ist Pantothensäure auxotroph und zeigt bei Supplementation mit β-Alanin und D-Pantoat kein Wachstum.

Zur Bestimmung von Pantothenat mit diesem Indikatorstamm wurde CGXII-Medium (Keilhauer et al., Journal of Bacteriology (1993) 175: 5595-5603) als Testmedium eingesetzt. Dazu wurden je 3 ml 4/3 fach konzentriertes CGXII-Medium in einem Inkubationsröhrchen (Falcon 2057, Becton and Dickinson, New Jersey, USA) mit 1 ml Pantothensäure-haltiger, steriler Eich- oder Probelösung versetzt und mit dem Indikatorstamm inokuliert. Als Inokulum wurden jeweils 60 µl einer Glyzerinkultur des Indikatorstammes eingesetzt. Nach 40 stündiger Inkubation bei 30°C wurde die Zelldichte (OD₆₀₀) (Novaspec 4049 Spectrophotometer, LKB Biochrom,Cambridge, GB) der Testansätze bestimmt und mittels einer Eichkurve die Pantothensäurekonzentration ermittelt. Der Stamm weist bis zu einer Konzentration von 25 µg/l eine lineare Abhängigkeit des Wachstums von der Pantothenatkonzentration auf, bei einer optischen Dichte von 0,5 bis 10. Zur Herstellung der Glyzerinkultur des Indikatorstammes wurde dieser Stamm auf unsupplementiertem CGXII-Medium 24 Stunden inkubiert (Aushungerung an D-Pantothenat). 1050 µl der Kultur wurden anschließend mit 700 µl Glyzerin versetzt. Von dieser bei -70°C zwischengefrorenen Glyzerinkultur wurden 60 µl zu Bestimmung von D-Pantothenat, wie zuvor beschrieben benutzt. Als Referenz wurde Na-Pantothenat verwendet, das von der Firma Sigma (Deisenhofen, Deutschland) bezogen wurde.

### Beispiel 7

### Produktion von D-Pantothenat mit verschiedenen C. glutamicum Stämmen

Zur Untersuchung ihrer Pantothenatbildung wurden die Stämme ATCC13032, ATCC13032/pEKEx2panBC, ATCC13032ΔilvA und ATCC13032ΔilvA/pEKEx2panBC in 60 ml Brain Heart Infusion-Medium (Difco Laboratories, Detroit, USA) für 14 Stunden bei 30°C vorkultiviert. Anschließend wurden die Zellen zweimal mit 0,9% NaCl-Lösung (w/v) gewaschen und mit dieser Suspension je 60 ml CgXII-Medium so angeimpft, daß die OD600 von 0,5 betrug. Das Medium war identisch mit dem bei Keilhauer et al., (Journal of Bacteriology (1993) 175: 5595-5603) beschriebenen Medium, enthielt aber zusätzlich 2 mM L-Isoleucin. Das von Keilhauer et al. beschriebene Medium CgXII ist in Tabelle 2 dargestellt.

**Tabelle 2**

| Zusammensetzung des Mediums CGXII | |
|---|---|
| Komponente | Konzentration |
| (NH₄)₂SO₄ | 20 g/L |
| Harnstoff | 5 g/L |
| KH₂PO₄ | 1 g/L |
| K₂HPO₄ | 1 g/L |
| Mg₂O₄∗7 H₂O | 0,25 g/L |
| 3-Morpholinopropansulfonsäure | 42 g/L |
| CaCl₂ | 10 mg/L |
| FeSO₄∗7 H₂O | 10 mg/L |
| MnSO₄∗ H₂O | 10 mg/L |
| ZnSO₄∗7 H₂O | 1 mg/L |
| CuSO₄ | 0,2 mg/L |
| NiCl₂∗6 H₂O | 0,02 mg/L |
| Biotin (pH7) | 0,2 mg/L |
| Glukose | 40 g/L |
| Protokatechusäure | 0,03 mg/L |

Bei der Kultivierung der Stämme ATCC13032/pEKEx2panBC und Stammes ATCC13032ΔilvA/pEKEx2panBC wurde das Medium nach 5 Stunden zusätzlich mit 1 mM Isopropylthio-β-D-galactosid versetzt. Nach 24 stündiger Kultivierung wurden Proben genommen, die Zellen abzentrifugiert und der Überstand sterilfiltriert. Die Pantothenatkonzentration des Überstands wurde mit Hilfe des im Beispiel 6 beschriebenen Pantothenattests bestimmt. Die Ergebnisse sind in Tabelle 3 dargestellt.

**Tabelle 3**

| D-Pantothenatbildung in verschiedenen C. glutamicum Stämmen | |
|---|---|
| Stamm | D-Pantothenat (mg/l) |
| ATCC13032 | 0,01 |
| ATCC13032/pEKEx2panBC | 0,03 |
| ATCC13032ΔilvA | 0,06 |
| ATCC13032ΔilvA/pEKEx2panBC | 0,3 |

### Beispiel 9

### Produktion von D-Pantothenat mit verschiedenen C. glutamicum Stämmen bei β-Alanin Zugabe

Zur Quantifizierung der Pantothenatbildung wurden die Stämme ATCC13032ΔilvA/pECM3ilvBNCD pEKEx2 und ATCC13032ΔilvA/pECM3ilvBNCD pEKEx2panBC in 60 ml Brain Heart Infusion-Medium(Difco Laboratories, Detroit, USA) mit 25 mg/l Kanamycin und 3 mg/l Chloramphenicol für 14 Stunden bei 30°C vorkultiviert, zweimal mit 0,9% NaCl-Lösung (w/v) gewaschen und mit dieser Suspension je 60 ml CgXII-Medium so angeimpft, daß die OD600 0,5 betrug. Das Medium enthielt hierbei 2 mM L-Isoleucin, 25 mg/l Kanamycin, 3 mg/l Chloramphenicol und β-Alanin in einer Endkonzentration von 20 mM. Nach 5 stündiger Kultivierung wurde dem Medium jeweils IPTG (Isopropylthio-β-D-galactosid) in einer Endkonzentration von 1 mM zugefügt. Nach 49 und 74 Stunden wurde eine Probe entnommen, die Zellen wurden abzentrifugiert und der Überstand sterilfiltriert. Die Pantothenatkonzentration des Überstands wurde wie in Beispiel 6 beschrieben bestimmt. Die Ergebnisse sind in Tabelle 4 dargestellt.

**Tabelle 4**

| D-Pantothenatakkumulation in verschiedenen Stämmen von C. glutamicum | | |
|---|---|---|
| Stamm | D-Pantothenat [mg/l] nach einer Inkubationszeit von | |
| | 49 Stunden | 74 Stunden |
| ATCC13032ΔilvA/pECM3ilvBNCD pEKEx2 | 80 | 100 |
| ATCC13032ΔilvA/pECM3ilvBNCD pEKEx2panBC | 920 | 980 |

SEQUENZPROTOKOLL

(1) ALLGEMEINE ANGABEN:

(i) ANMELDER:
(A) NAME: Degussa Aktiengesellschaft
(B) STRASSE: Weissfrauenstr. 9
(C) ORT: Frankfurt am Main
(D) BUNDESLAND: Hessen
(E) LAND: Deutschland
(F) POSTLEITZAHL: D-60311

(A) NAME: Forschungszentrum Juelich GmbH
(B) STRASSE: Leo-Brandt Strasse
(C) ORT: Juelich
(D) BUNDESLAND: Nordrhein-Westfalen
(E) LAND: Deutschland
(F) POSTLEITZAHL: D-52425

(ii) BEZEICHNUNG DER ERFINDUNG: Verfahren zur
fermentativen Herstellung von D-Pantothensaeure
unter Verwendung coryneformer Bakterien

(iii) ANZAHL DER SEQUENZEN: 5

(iv) COMPUTER-LESBARE FASSUNG:
(A) DATENTRAEGER: Floppy disk
(B) COMPUTER: IBM PC compatible
(C) BETRIEBSSYSTEM: PC-DOS/MS-DOS
(D) SOFTWARE: PatentIn Release #1.0, Version
#1.30 (EPA)

(2) ANGABEN ZU SEQ ID NO: 1:

(i) SEQUENZKENNZEICHEN:
(A) LAENGE: 2164 Basenpaare
(B) ART: Nucleotid
(C) STRANGFORM: Doppelstrang
(D) TOPOLOGIE: linear

(ii) ART DES MOLEKUELS: Genom-DNA

(iii) HYPOTHETISCH: NEIN

(iv) ANTISENSE: NEIN

(vi) URSPRUENGLICHE HERKUNFT:
(A) ORGANISMUS: Corynebacterium glutamicum
(B) STAMM: ATCC13032

(ix) MERKMAL:
(A) NAME/SCHLUESSEL: CDS
(B) LAGE:351..1163
(D) SONSTIGE ANGABEN:/codon_start= 351
/EC_number= 4.1.2.12
/product=
"Ketopantoathydroxymethyltransferase"
/gene= "panB"

(ix) MERKMAL:
(A) NAME/SCHLUESSEL: CDS
(B) LAGE:1166..2002
(D) SONSTIGE ANGABEN:/codon_start= 1166
/EC_number= 6.3.2.1
/product= "Pantothenatsynthetase"
/gene= "panC"

(xi) SEQUENZBESCHREIBUNG: SEQ ID NO: 1:

GCTTCGGGGT ACCAATTCCT TTAAGAACCA TCAGATCAAT CTGTTGTACA TTCTCGGCCA 60

GATTCAGCTT TTCGGTAAGG ACGAAACACT TTCACTTGAA TCGGCAGCAA AGTTTCTTAA 120

AGTTTCTAAG GCAACTGCAA CGAGGTATTT TAGAACTCTC CGAGAAATGG AATTAGTTCA 180

CGAGGTCAGC AAACGCCCTT TGCGGTTTGC GCTCACGGAT AAAGGTCGTG AGATAGTAGG 240

TCTTGAGGTA AAAATTTGAC TCCATAACGA GAACTTAATC GAGCAACACC CCTGAACAGT 300

GAATCAAATC GGAATTTATT TATTCTGAGC TGGTCATCAC ATCTATACTC ATG CCC 356
Met Pro
1

ATG TCA GGC ATT GAT GCA AAG AAA ATC CGC ACC CGT CAT TTC CGC GAA 404
Met Ser Gly Ile Asp Ala Lys Lys Ile Arg Thr Arg His Phe Arg Glu
        5                   10                  15

GCT AAA GTA AAC GGC CAG AAA GTT TCG GTT CTC ACC AGC TAT GAT GCG 452
Ala Lys Val Asn Gly Gln Lys Val Ser Val Leu Thr Ser Tyr Asp Ala
    20                  25                  30

CTT TCG GCG CGC ATT TTT GAT GAG GCT GGC GTC GAT ATG CTC CTT GTT 500
Leu Ser Ala Arg Ile Phe Asp Glu Ala Gly Val Asp Met Leu Leu Val
35                  40                  45                  50

GGT GAT TCC GCT GCC AAC GTT GTG CTG GGT CGC GAT ACC ACC TTG TCG 548
Gly Asp Ser Ala Ala Asn Val Val Leu Gly Arg Asp Thr Thr Leu Ser
                55                  60                  65

ATC ACC TTG GAT GAG ATG ATT GTG CTG GCC AAG GCG GTG ACG ATC GCT 596
Ile Thr Leu Asp Glu Met Ile Val Leu Ala Lys Ala Val Thr Ile Ala
            70                  75                  80

ACG AAG CGT GCG CTT GTG GTG GTT GAT CTG CCG TTT GGT ACC TAT GAG 644
Thr Lys Arg Ala Leu Val Val Val Asp Leu Pro Phe Gly Thr Tyr Glu
        85                  90                  95

GTG AGC CCA AAT CAG GCG GTG GAG TCC GCG ATC CGG GTC ATG CGT GAA 692
Val Ser Pro Asn Gln Ala Val Glu Ser Ala Ile Arg Val Met Arg Glu
    100                 105                 110

ACG GGT GCG GCT GCG GTG AAG ATC GAG GGT GGC GTG GAG ATC GCG CAG 740

Thr Gly Ala Ala Ala Val Lys Ile Glu Gly Gly Val Glu Ile Ala Gln
115                 120                 125                 130

ACG ATT CGA CGC ATT GTT GAT GCT GGA ATT CCG GTT GTC GGC CAC ATC      788
Thr Ile Arg Arg Ile Val Asp Ala Gly Ile Pro Val Val Gly His Ile
                135                 140                 145

GGG TAC ACC CCG CAG TCC GAG CAT TCC TTG GGC GGC CAC GTG GTT CAG      836
Gly Tyr Thr Pro Gln Ser Glu His Ser Leu Gly Gly His Val Val Gln
            150                 155                 160

GGT CGT GGC GCG AGT TCT GGA AAG CTC ATC GCC GAT GCC CGC GCG TTG      884
Gly Arg Gly Ala Ser Ser Gly Lys Leu Ile Ala Asp Ala Arg Ala Leu
        165                 170                 175

GAG CAG GCG GGT GCG TTT GCG GTT GTG TTG GAG ATG GTT CCA GCA GAG      932
Glu Gln Ala Gly Ala Phe Ala Val Val Leu Glu Met Val Pro Ala Glu
    180                 185                 190

GCA GCG CGC GAG GTT ACC GAG GAT CTT TCC ATC ACC ACT ATC GGA ATC      980
Ala Ala Arg Glu Val Thr Glu Asp Leu Ser Ile Thr Thr Ile Gly Ile
195                 200                 205                 210

GGT GCC GGC AAT GGC ACA GAT GGG CAG GTT TTG GTG TGG CAG GAT GCC     1028
Gly Ala Gly Asn Gly Thr Asp Gly Gln Val Leu Val Trp Gln Asp Ala
                215                 220                 225

TTC GGC CTC AAC CGC GGC AAG AAG CCA CGC TTC GTC CGC GAG TAC GCC     1076
Phe Gly Leu Asn Arg Gly Lys Lys Pro Arg Phe Val Arg Glu Tyr Ala
            230                 235                 240

ACC TTG GGC GAT TCC TTG CAC GAC GCC GCG CAG GCC TAC ATC GCC GAT     1124
Thr Leu Gly Asp Ser Leu His Asp Ala Ala Gln Ala Tyr Ile Ala Asp
        245                 250                 255

ATC CAC GCG GGT ACC TTC CCA GGC GAA GCG GAG TCC TTT TA ATG CAG      1171
Ile His Ala Gly Thr Phe Pro Gly Glu Ala Glu Ser Phe    Met Gln
    260                 265                 270                 1

GTA GCA ACC ACA AAG CAG GCG CTT ATC GAC GCC CTC CTC CAC CAC AAA     1219
Val Ala Thr Thr Lys Gln Ala Leu Ile Asp Ala Leu Leu His His Lys
            5                   10                  15

TCC GTC GGG CTC GTC CCC ACC ATG GGT GCG CTA CAC AGC GGA CAC GCC     1267
Ser Val Gly Leu Val Pro Thr Met Gly Ala Leu His Ser Gly His Ala
    20                  25                  30

TCG TTG GTT AAA GCA GCA CGC GCT GAA AAC GAC ACT GTT GTA GCC AGT     1315
Ser Leu Val Lys Ala Ala Arg Ala Glu Asn Asp Thr Val Val Ala Ser
35                  40                  45                  50

ATT TTT GTC AAT CCC CTG CAG TTT GAA GCA CTC GGT GAT TGC GAT GAT     1363
Ile Phe Val Asn Pro Leu Gln Phe Glu Ala Leu Gly Asp Cys Asp Asp
                55                  60                  65

TAC CGC AAC TAT CCC CGC CAA CTC GAC GCC GAT TTA GCA CTG CTT GAA     1411
Tyr Arg Asn Tyr Pro Arg Gln Leu Asp Ala Asp Leu Ala Leu Leu Glu
            70                  75                  80

GAG GCA GGT GTG GAT ATT GTG TTC GCA CCC GAT GTG GAG GAA ATG TAC     1459
Glu Ala Gly Val Asp Ile Val Phe Ala Pro Asp Val Glu Glu Met Tyr
        85                  90                  95

CCC GGT GGC TTG CCA CTA GTG TGG GCG CGC ACC GGT TCC ATC GGA ACA     1507
Pro Gly Gly Leu Pro Leu Val Trp Ala Arg Thr Gly Ser Ile Gly Thr
    100                 105                 110

AAA TTG GAG GGT GCC AGC AGG CCT GGC CAT TTC GAT GGT GTG GCT ACC 1555
Lys Leu Glu Gly Ala Ser Arg Pro Gly His Phe Asp Gly Val Ala Thr
115 120 125 130

GTG GTG GCG AAG CTG TTC AAT TTG GTG CGC CCT GAT CGT GCA TAT TTT 1603
Val Val Ala Lys Leu Phe Asn Leu Val Arg Pro Asp Arg Ala Tyr Phe
135 140 145

GGA CAA AAA GAT GCT CAG CAG GTT GCG GTG ATT CGG CGA TTG GTT GCC 1651
Gly Gln Lys Asp Ala Gln Gln Val Ala Val Ile Arg Arg Leu Val Ala
150 155 160

GAT CTA GAC ATT CCC GTG GAG ATT CGT CCC GTT CCG ATT ATT CGT GGC 1699
Asp Leu Asp Ile Pro Val Glu Ile Arg Pro Val Pro Ile Ile Arg Gly
165 170 175

GCC GAT GGC TTA GCC GAA TCC AGC CGC AAT CAA CGT CTT TCT GCG GAT 1747
Ala Asp Gly Leu Ala Glu Ser Ser Arg Asn Gln Arg Leu Ser Ala Asp
180 185 190

CAG CGA GCG CAA GCT CTG GTG CTG CCG CAG GTG TTG AGT GGG TTG CAG 1795
Gln Arg Ala Gln Ala Leu Val Leu Pro Gln Val Leu Ser Gly Leu Gln
195 200 205 210

CGT CGA AAA GCA GCT GGT GAA GCG CTA GAT ATC CAA GGT GCG CGC GAC 1843
Arg Arg Lys Ala Ala Gly Glu Ala Leu Asp Ile Gln Gly Ala Arg Asp
215 220 225

ACC TTG GCC AGC GCC GAC GGC GTG CGC TTG GAT CAC CTG GAA ATT GTC 1891
Thr Leu Ala Ser Ala Asp Gly Val Arg Leu Asp His Leu Glu Ile Val
230 235 240

GAT CCA GCC ACC CTC GAA CCA TTA GAA ATC GAC GGC CTG CTC ACC CAA 1939
Asp Pro Ala Thr Leu Glu Pro Leu Glu Ile Asp Gly Leu Leu Thr Gln
245 250 255

CCA GCG TTG GTG GTC GGC GCG ATT TTC GTG GGG CCG GTG CGG TTG ATC 1987
Pro Ala Leu Val Val Gly Ala Ile Phe Val Gly Pro Val Arg Leu Ile
260 265 270

GAC AAT ATC GAG CTC TAGTACCAAC CCTGCGTTGC AGCACGCAGC TTCGCATAAC 2042
Asp Asn Ile Glu Leu
275

GCGTGCTCAG CTCAGTGTTT TTAGGTGCGC GGTGCGGATC GGAACCGGGA GTTGGCCACT 2102

GCGGTGGCGT GGCCTCACCC GACAGCGCCC ATGCCGCCTG ACGAGCTGCA CCCAACGCCA 2162

CA 2164

(2) ANGABEN ZU SEQ ID NO: 2:

(i) SEQUENZKENNZEICHEN:
(A) LAENGE: 271 Aminosaeuren
(B) ART: Aminosaeure
(D) TOPOLOGIE: linear

(ii) ART DES MOLEKUELS: Protein
(xi) SEQUENZBESCHREIBUNG: SEQ ID NO: 2:

Met Pro Met Ser Gly Ile Asp Ala Lys Lys Ile Arg Thr Arg His Phe
1               5                   10                  15

Arg Glu Ala Lys Val Asn Gly Gln Lys Val Ser Val Leu Thr Ser Tyr
            20                  25                  30

Asp Ala Leu Ser Ala Arg Ile Phe Asp Glu Ala Gly Val Asp Met Leu

        35                  40                  45

Leu Val Gly Asp Ser Ala Ala Asn Val Val Leu Gly Arg Asp Thr Thr
    50                  55                  60

Leu Ser Ile Thr Leu Asp Glu Met Ile Val Leu Ala Lys Ala Val Thr
65                  70                  75                  80

Ile Ala Thr Lys Arg Ala Leu Val Val Val Asp Leu Pro Phe Gly Thr
                85                  90                  95

Tyr Glu Val Ser Pro Asn Gln Ala Val Glu Ser Ala Ile Arg Val Met
            100                 105                 110

Arg Glu Thr Gly Ala Ala Ala Val Lys Ile Glu Gly Gly Val Glu Ile
        115                 120                 125

Ala Gln Thr Ile Arg Arg Ile Val Asp Ala Gly Ile Pro Val Val Gly
    130                 135                 140

His Ile Gly Tyr Thr Pro Gln Ser Glu His Ser Leu Gly Gly His Val
145                 150                 155                 160

Val Gln Gly Arg Gly Ala Ser Ser Gly Lys Leu Ile Ala Asp Ala Arg
                165                 170                 175

Ala Leu Glu Gln Ala Gly Ala Phe Ala Val Val Leu Glu Met Val Pro
            180                 185                 190

Ala Glu Ala Ala Arg Glu Val Thr Glu Asp Leu Ser Ile Thr Thr Ile
        195                 200                 205

Gly Ile Gly Ala Gly Asn Gly Thr Asp Gly Gln Val Leu Val Trp Gln
    210                 215                 220

Asp Ala Phe Gly Leu Asn Arg Gly Lys Lys Pro Arg Phe Val Arg Glu
225                 230                 235                 240

Tyr Ala Thr Leu Gly Asp Ser Leu His Asp Ala Ala Gln Ala Tyr Ile
                245                 250                 255

Ala Asp Ile His Ala Gly Thr Phe Pro Gly Glu Ala Glu Ser Phe
            260                 265                 270

(2) ANGABEN ZU SEQ ID NO: 3:

(i) SEQUENZKENNZEICHEN:
(A) LAENGE: 279 Aminosaeuren
(B) ART: Aminosaeure
(D) TOPOLOGIE: linear

(ii) ART DES MOLEKUELS: Protein
(xi) SEQUENZBESCHREIBUNG: SEQ ID NO: 3:

Met Gln Val Ala Thr Thr Lys Gln Ala Leu Ile Asp Ala Leu Leu His
1               5                   10                  15

His Lys Ser Val Gly Leu Val Pro Thr Met Gly Ala Leu His Ser Gly
            20                  25                  30

His Ala Ser Leu Val Lys Ala Ala Arg Ala Glu Asn Asp Thr Val Val
        35                  40                  45

Ala Ser Ile Phe Val Asn Pro Leu Gln Phe Glu Ala Leu Gly Asp Cys
    50                  55                  60

Asp Asp Tyr Arg Asn Tyr Pro Arg Gln Leu Asp Ala Asp Leu Ala Leu

65                  70                  75                  80

Leu Glu Glu Ala Gly Val Asp Ile Val Phe Ala Pro Asp Val Glu Glu
                85                  90                  95

Met Tyr Pro Gly Gly Leu Pro Leu Val Trp Ala Arg Thr Gly Ser Ile
            100                 105                 110

Gly Thr Lys Leu Glu Gly Ala Ser Arg Pro Gly His Phe Asp Gly Val
        115                 120                 125

Ala Thr Val Val Ala Lys Leu Phe Asn Leu Val Arg Pro Asp Arg Ala
    130                 135                 140

Tyr Phe Gly Gln Lys Asp Ala Gln Gln Val Ala Val Ile Arg Arg Leu
145                 150                 155                 160

Val Ala Asp Leu Asp Ile Pro Val Glu Ile Arg Pro Val Pro Ile Ile
                165                 170                 175

Arg Gly Ala Asp Gly Leu Ala Glu Ser Ser Arg Asn Gln Arg Leu Ser
            180                 185                 190

Ala Asp Gln Arg Ala Gln Ala Leu Val Leu Pro Gln Val Leu Ser Gly
        195                 200                 205

Leu Gln Arg Arg Lys Ala Ala Gly Glu Ala Leu Asp Ile Gln Gly Ala
    210                 215                 220

Arg Asp Thr Leu Ala Ser Ala Asp Gly Val Arg Leu Asp His Leu Glu
225                 230                 235                 240

Ile Val Asp Pro Ala Thr Leu Glu Pro Leu Glu Ile Asp Gly Leu Leu
                245                 250                 255

Thr Gln Pro Ala Leu Val Val Gly Ala Ile Phe Val Gly Pro Val Arg
            260                 265                 270

Leu Ile Asp Asn Ile Glu Leu
        275

(2) ANGABEN ZU SEQ ID NO: 4:

     (i) SEQUENZKENNZEICHEN:
          (A) LAENGE: 2952 Basenpaare
          (B) ART: Nucleotid
          (C) STRANGFORM: Doppelstrang
          (D) TOPOLOGIE: linear

    (ii) ART DES MOLEKUELS: Genom-DNA

   (iii) HYPOTHETISCH: NEIN

    (iv) ANTISENSE: NEIN

    (vi) URSPRUENGLICHE HERKUNFT:
          (A) ORGANISMUS: Corynebacterium glutamicum
          (B) STAMM: ATCC13032
          (C) INDIVIDUUM/ISOLAT: MUTANTE R127

    (ix) MERKMAL:
          (A) NAME/SCHLUESSEL: CDS
          (B) LAGE:290..2125
          (D) SONSTIGE ANGABEN:/codon_start= 290
               /EC_number= 4.2.1.9
               /product= "Dihydroxysaeuredehydratase"
               /gene= "ilvD"

(xi) SEQUENZBESCHREIBUNG: SEQ ID NO: 4:

AGTACTTGGA GCGCCAAAAG GCACTGGGCA AGCCAGTTCA GTTGAACTTC GATGACGACA 60

CCGATGGGAA TACAACACAA ACAGAAAGCG TTGAATCCCA AGAGACCGGA CAAGCCGCGT 120

CTGAAACCTC ACATCGTGAT AACCCTGCGT CACAGCACTA GAGTGTAATA AGCCGTCCGA 180

ACCAAAGGTC CACACCTCTG CACGAGTAGA AGCTCACCCA AGTTTTCAAA GTGCCGTTGA 240

TTCTTGACAA CCACCCGCCG CTCTTTAGAG CAGATTTGAA AAGCGCATC ATG ATC 295
Met Ile
280

CCA CTT CGT TCA AAA GTC ACC ACC GTC GGT CGC AAT GCA GCT GGC GCT 343
Pro Leu Arg Ser Lys Val Thr Thr Val Gly Arg Asn Ala Ala Gly Ala
285 290 295

CGC GCC CTT TGG CGT GCC ACC GGC ACC AAG GAA AAT GAG TTC GGC AAG 391
Arg Ala Leu Trp Arg Ala Thr Gly Thr Lys Glu Asn Glu Phe Gly Lys
300 305 310

CCA ATT GTT GCC ATC GTA AAC TCC TAC ACC CAG TTC GTG CCC GGA CAC 439
Pro Ile Val Ala Ile Val Asn Ser Tyr Thr Gln Phe Val Pro Gly His
315 320 325

GTT CAC CTT AAG AAC GTC GGC GAT ATT GTG GCA GAT GCA GTG CGC AAA 487
Val His Leu Lys Asn Val Gly Asp Ile Val Ala Asp Ala Val Arg Lys
330 335 340 345

GCC GGT GGC GTT CCA AAG GAA TTC AAC ACC ATC GTC GAT GAC GGC ATC 535
Ala Gly Gly Val Pro Lys Glu Phe Asn Thr Ile Val Asp Asp Gly Ile
350 355 360

GCC ATG GGA CAC GGC GGC ATG CTG TAC TCC CTG CCA TCC CGT GAA ATC 583
Ala Met Gly His Gly Gly Met Leu Tyr Ser Leu Pro Ser Arg Glu Ile
365 370 375

ATC GCC GAC TCC GTC GAA TAC ATG GTC AAC GCA CAC ACC GCC GAC GCC 631
Ile Ala Asp Ser Val Glu Tyr Met Val Asn Ala His Thr Ala Asp Ala
380 385 390

ATG GTG TGT ATC TCC AAC TGT GAC AAG ATC ACC CCA GGC ATG CTC AAC 679
Met Val Cys Ile Ser Asn Cys Asp Lys Ile Thr Pro Gly Met Leu Asn
395 400 405

GCA GCA ATG CGC CTG AAC ATC CCA GTG GTC TTC GTT TCC GGT GGC CCA 727
Ala Ala Met Arg Leu Asn Ile Pro Val Val Phe Val Ser Gly Gly Pro
410 415 420 425

ATG GAA GCT GGC AAG GCT GTC GTC GTT GAG CGC GTT GCA CAC GCA CCA 775
Met Glu Ala Gly Lys Ala Val Val Val Glu Arg Val Ala His Ala Pro
430 435 440

ACC GAC CTC ATC ACC GCG ATC TCC GCA TCC GCA AGC GAT GCA GTC GAC 823
Thr Asp Leu Ile Thr Ala Ile Ser Ala Ser Ala Ser Asp Ala Val Asp
445 450 455

GAC GCA GGC CTT GCA GCC GTT GAA CGA TCC GCA TGC CCA ACC TGT GGC 871
Asp Ala Gly Leu Ala Ala Val Glu Arg Ser Ala Cys Pro Thr Cys Gly
460 465 470

TCC TGC TCC GGT ATG TTC ACC GCG AAC TCC ATG AAC TGC CTC ACC GAA 919
Ser Cys Ser Gly Met Phe Thr Ala Asn Ser Met Asn Cys Leu Thr Glu
475 480 485

GCT CTG GGA CTT TCT CTC CCG GGC AAC GGC TCC ACT CTG GCA ACC CAC 967

Ala Leu Gly Leu Ser Leu Pro Gly Asn Gly Ser Thr Leu Ala Thr His
490                 495                 500                 505

GCA GCA CGT CGC GCA CTG TTT GAA AAG GCC GGC GAA ACC GTC GTT GAA        1015
Ala Ala Arg Arg Ala Leu Phe Glu Lys Ala Gly Glu Thr Val Val Glu
                510                 515                 520

CTG TGC CGC CGC TAC TAC GGT GAA GAA GAC GAA TCC GTT CTG CCA CGT        1063
Leu Cys Arg Arg Tyr Tyr Gly Glu Glu Asp Glu Ser Val Leu Pro Arg
            525                 530                 535

GGC ATT GCC ACC AAG AAG GCA TTC GAA AAC GCA ATG GCA CTG GAT ATG        1111
Gly Ile Ala Thr Lys Lys Ala Phe Glu Asn Ala Met Ala Leu Asp Met
        540                 545                 550

GCC ATG GGT GGA TCC ACC AAC ACC ATC CTC CAC ATC CTC GCA GCT GCC        1159
Ala Met Gly Gly Ser Thr Asn Thr Ile Leu His Ile Leu Ala Ala Ala
    555                 560                 565

CAG GAA GGC GAA GTT GAC TTC GAC CTC GCA GAC ATC GAC GAA CTG TCC        1207
Gln Glu Gly Glu Val Asp Phe Asp Leu Ala Asp Ile Asp Glu Leu Ser
570                 575                 580                 585

AAA AAC GTC CCC TGC CTG TCC AAG GTT GCA CCA AAC TCC GAC TAC CAC        1255
Lys Asn Val Pro Cys Leu Ser Lys Val Ala Pro Asn Ser Asp Tyr His
                590                 595                 600

ATG GAA GAC GTC CAC CGC GCC GGT CGC ATT CCA GCA CTG CTC GGC GAG        1303
Met Glu Asp Val His Arg Ala Gly Arg Ile Pro Ala Leu Leu Gly Glu
            605                 610                 615

CTC AAC CGC GGT GGC CTG CTG AAC AAG GAC GTC CAC TCC GTT CAC TCC        1351
Leu Asn Arg Gly Gly Leu Leu Asn Lys Asp Val His Ser Val His Ser
        620                 625                 630

AAC GAC CTT GAA GGT TGG TTG GAT GAC TGG GAT ATC CGC TCT GGC AAG        1399
Asn Asp Leu Glu Gly Trp Leu Asp Asp Trp Asp Ile Arg Ser Gly Lys
    635                 640                 645

ACC ACC GAA GTA GCA ACC GAA CTC TTC CAC GCA GCC CCA GGT GGC ATC        1447
Thr Thr Glu Val Ala Thr Glu Leu Phe His Ala Ala Pro Gly Gly Ile
650                 655                 660                 665

CGC ACC ACC GAA GCA TTC TCC ACC GAG AAC CGC TGG GAC GAA CTC GAC        1495
Arg Thr Thr Glu Ala Phe Ser Thr Glu Asn Arg Trp Asp Glu Leu Asp
                670                 675                 680

ACC GAC GCT GCC AAG GGC TGC ATC CGC GAC GTT GAA CAC GCC TAC ACC        1543
Thr Asp Ala Ala Lys Gly Cys Ile Arg Asp Val Glu His Ala Tyr Thr
            685                 690                 695

GCC GAC GGC GGC CTG GTT GTT CTT CGC GGC AAC ATC TCC CCT GAC GGC        1591
Ala Asp Gly Gly Leu Val Val Leu Arg Gly Asn Ile Ser Pro Asp Gly
        700                 705                 710

GCA GTG ATC AAG TCC GCA GGT ATC GAA GAA GAG CTG TGG AAC TTC ACC        1639
Ala Val Ile Lys Ser Ala Gly Ile Glu Glu Glu Leu Trp Asn Phe Thr
    715                 720                 725

GGA CCA GCA CGA GTT GTC GAA AGC CAG GAA GAG GCA GTC TCT GTC ATC        1687
Gly Pro Ala Arg Val Val Glu Ser Gln Glu Glu Ala Val Ser Val Ile
730                 735                 740                 745

CTG ACC AAG ACC ATC CAA GCT GGC GAA GTT CTG GTC GTC CGC TAC GAA        1735
Leu Thr Lys Thr Ile Gln Ala Gly Glu Val Leu Val Val Arg Tyr Glu
                750                 755                 760

GGC CCA TCA GGT GGA CCA GGC ATG CAG GAA ATG CTT CAC CCA ACC GCA        1783

Gly Pro Ser Gly Gly Pro Gly Met Gln Glu Met Leu His Pro Thr Ala
765 770 775

TTC CTC AAG GGA TCC GGC CTG GGC AAG AAG TGT GCA CTG ATC ACC GAC 1831
Phe Leu Lys Gly Ser Gly Leu Gly Lys Lys Cys Ala Leu Ile Thr Asp
780 785 790

GGC CGT TTC TCC GGA GGT TCC TCA GGA CTG TCC ATC GGC CAC GTC TCC 1879
Gly Arg Phe Ser Gly Gly Ser Ser Gly Leu Ser Ile Gly His Val Ser
795 800 805

CCA GAA GCA GCA CAC GGC GGA GTC ATT GGT CTG ATC GAA AAC GGC GAC 1927
Pro Glu Ala Ala His Gly Gly Val Ile Gly Leu Ile Glu Asn Gly Asp
810 815 820 825

ATC GTC TCC ATC GAC GTT CAC AAC CGC AAG CTC GAA GTT CAG GTC TCC 1975
Ile Val Ser Ile Asp Val His Asn Arg Lys Leu Glu Val Gln Val Ser
830 835 840

GAC GAG GAA CTC CAG CGC CGC CGC GAC GCT ATG AAC GCC TCC GAG AAG 2023
Asp Glu Glu Leu Gln Arg Arg Arg Asp Ala Met Asn Ala Ser Glu Lys
845 850 855

CCA TGG CAG CCA GTC AAC CGT AAC CGC GTT GTC ACC AAG GCA CTG CGC 2071
Pro Trp Gln Pro Val Asn Arg Asn Arg Val Val Thr Lys Ala Leu Arg
860 865 870

GCA TAC GCA AAG ATG GCT ACC TCC GCT GAT AAG GGT GCA GTC CGT CAG 2119
Ala Tyr Ala Lys Met Ala Thr Ser Ala Asp Lys Gly Ala Val Arg Gln
875 880 885

GTC GAC TAACCCTTTG TGAGTGTTTG AGCACCGGTT CCCTACTTTG GGTTCCGGTG 2175
Val Asp
890

CTTTTTCATG TCTTGGCCTG TGTGGGCGTG GTGGAGCTCC CCGTTGCAAA TACTCACCAC 2235

AAGTTGCAGG ATTTCTGCTG GTTGTGGTGG ATTTTCCCGC TTTATAGCCC TATGCGTGCA 2295

ACTTTCGGAC CGATTCCAAA GGGCAAAGCC CTGTTTGTGG TGGATCCTTG CCCTGGAAGC 2355

TTTCAGGAAC CACAACTACC CCACTGACCC CAAAGTGGAT AGGCCCTATT CTTCCGTTTA 2415

AGCGCCTCAA ACACCTCTCC CCACACTTGA CCCATTAGGC AATTACGAAT CCTTAAACAG 2475

CCTTCTACAG CACCATGCCC CAAACCGAAC CCAGGCATGA AAAAGACCCT CACCAGGAGG 2535

GTCTTTTTCT AAAACTTTGG CTACGCGATT GGGTTCACAC CCGCACCGAA CCACCACAGC 2595

AGAACTGCCG CTGCGATGCC GATGACCACG AAGATCCACG AGCTCACCAG TGGACGCTTT 2655

GCCCAACCTC GGCCAGAGTC AAGGGAAATC TTGCCGGGGC CGGTGAACTG AAGTCCGACA 2715

ACCACGATAG TGAGGATCAG TGCCAGCATC AATGGCTCAC TAAGTTCACC CCAACCACCT 2775

TCATGAGTGT TGACTTGGTG AAGGGTGGTA AAGGATGTCG CCACCGTGGC TACCGCTGCT 2835

GCCACTGGGG TCATCAGACC AAGGAGCAGG AAGACACCAG CCGCAAGTTC AATAGATGGA 2895

AGCAGGATCG CGAGGATTTC AGGCCACTGG TAACCAGCGA ACTCTGCCTC GACTCTA 2952

(2) ANGABEN ZU SEQ ID NO: 5:

(i) SEQUENZKENNZEICHEN:
(A) LAENGE: 612 Aminosaeuren
(B) ART: Aminosaeure
(D) TOPOLOGIE: linear

(ii) ART DES MOLEKUELS: Protein
(xi) SEQUENZBESCHREIBUNG: SEQ ID NO: 5:

Met Ile Pro Leu Arg Ser Lys Val Thr Thr Val Gly Arg Asn Ala Ala
1               5                   10                  15

Gly Ala Arg Ala Leu Trp Arg Ala Thr Gly Thr Lys Glu Asn Glu Phe
            20                  25                  30

Gly Lys Pro Ile Val Ala Ile Val Asn Ser Tyr Thr Gln Phe Val Pro
        35                  40                  45

Gly His Val His Leu Lys Asn Val Gly Asp Ile Val Ala Asp Ala Val
    50                  55                  60

Arg Lys Ala Gly Gly Val Pro Lys Glu Phe Asn Thr Ile Val Asp Asp
65                  70                  75                  80

Gly Ile Ala Met Gly His Gly Gly Met Leu Tyr Ser Leu Pro Ser Arg
                85                  90                  95

Glu Ile Ile Ala Asp Ser Val Glu Tyr Met Val Asn Ala His Thr Ala
            100                 105                 110

Asp Ala Met Val Cys Ile Ser Asn Cys Asp Lys Ile Thr Pro Gly Met
        115                 120                 125

Leu Asn Ala Ala Met Arg Leu Asn Ile Pro Val Val Phe Val Ser Gly
    130                 135                 140

Gly Pro Met Glu Ala Gly Lys Ala Val Val Val Glu Arg Val Ala His
145                 150                 155                 160

Ala Pro Thr Asp Leu Ile Thr Ala Ile Ser Ala Ser Ala Ser Asp Ala
                165                 170                 175

Val Asp Asp Ala Gly Leu Ala Ala Val Glu Arg Ser Ala Cys Pro Thr
            180                 185                 190

Cys Gly Ser Cys Ser Gly Met Phe Thr Ala Asn Ser Met Asn Cys Leu
        195                 200                 205

Thr Glu Ala Leu Gly Leu Ser Leu Pro Gly Asn Gly Ser Thr Leu Ala
    210                 215                 220

Thr His Ala Ala Arg Arg Ala Leu Phe Glu Lys Ala Gly Glu Thr Val
225                 230                 235                 240

Val Glu Leu Cys Arg Arg Tyr Tyr Gly Glu Glu Asp Glu Ser Val Leu
                245                 250                 255

Pro Arg Gly Ile Ala Thr Lys Lys Ala Phe Glu Asn Ala Met Ala Leu
            260                 265                 270

Asp Met Ala Met Gly Gly Ser Thr Asn Thr Ile Leu His Ile Leu Ala
        275                 280                 285

Ala Ala Gln Glu Gly Glu Val Asp Phe Asp Leu Ala Asp Ile Asp Glu
    290                 295                 300

Leu Ser Lys Asn Val Pro Cys Leu Ser Lys Val Ala Pro Asn Ser Asp
305                 310                 315                 320

Tyr His Met Glu Asp Val His Arg Ala Gly Arg Ile Pro Ala Leu Leu
                325                 330                 335

Gly Glu Leu Asn Arg Gly Gly Leu Leu Asn Lys Asp Val His Ser Val

340                 345                 350

His Ser Asn Asp Leu Glu Gly Trp Leu Asp Asp Trp Asp Ile Arg Ser
        355                 360                 365

Gly Lys Thr Thr Glu Val Ala Thr Glu Leu Phe His Ala Ala Pro Gly
    370                 375                 380

Gly Ile Arg Thr Thr Glu Ala Phe Ser Thr Glu Asn Arg Trp Asp Glu
385                 390                 395                 400

Leu Asp Thr Asp Ala Ala Lys Gly Cys Ile Arg Asp Val Glu His Ala
                405                 410                 415

Tyr Thr Ala Asp Gly Gly Leu Val Val Leu Arg Gly Asn Ile Ser Pro
            420                 425                 430

Asp Gly Ala Val Ile Lys Ser Ala Gly Ile Glu Glu Glu Leu Trp Asn
        435                 440                 445

Phe Thr Gly Pro Ala Arg Val Val Glu Ser Gln Glu Glu Ala Val Ser
    450                 455                 460

Val Ile Leu Thr Lys Thr Ile Gln Ala Gly Glu Val Leu Val Val Arg
465                 470                 475                 480

Tyr Glu Gly Pro Ser Gly Gly Pro Gly Met Gln Glu Met Leu His Pro
                485                 490                 495

Thr Ala Phe Leu Lys Gly Ser Gly Leu Gly Lys Lys Cys Ala Leu Ile
            500                 505                 510

Thr Asp Gly Arg Phe Ser Gly Gly Ser Ser Gly Leu Ser Ile Gly His
        515                 520                 525

Val Ser Pro Glu Ala Ala His Gly Gly Val Ile Gly Leu Ile Glu Asn
    530                 535                 540

Gly Asp Ile Val Ser Ile Asp Val His Asn Arg Lys Leu Glu Val Gln
545                 550                 555                 560

Val Ser Asp Glu Glu Leu Gln Arg Arg Arg Asp Ala Met Asn Ala Ser
                565                 570                 575

Glu Lys Pro Trp Gln Pro Val Asn Arg Asn Arg Val Val Thr Lys Ala
            580                 585                 590

Leu Arg Ala Tyr Ala Lys Met Ala Thr Ser Ala Asp Lys Gly Ala Val
        595                 600                 605

Arg Gln Val Asp
    610

### Abbildungen

Folgende Abbildungen sind beigefügt:
Abbildung 1:
   Restriktionskartierung von pUR1 und Lage des sequenzierten Fragments.
Abbildung 2:
   Restriktionskarte des Plasmids pEKEx2panBC.
Abbildung 3:
   Restriktionskarte des Plasmids pECM3ilvBNCD.

## Patentansprüche

1. In Mikroorganismen der Gattung Corynebacterium replizierbare, gegebenfalls rekombinante DNA mit der Herkunft Corynebacterium, die zumindest eine der folgenden Nucleotidsequenzen enthält, ausgewählt aus der Gruppe:
a) codierend für das panB-Gen (Ketopantoathydroxymethyltranferase), dargestellt in der SEQ-ID-No.1,
b) codierend für das panC-Gen (Pantothenatsynthetase), dargestellt in der SEQ-ID-No.1, insbesondere das panBC-Operon und gegebenenfalls
c) codierend für das ilvD-Gen (Dihydroxysäuredehydratase), dargestellt durch die SEQ-ID-No.4.

2. Replizierbare DNA gemäß Anspruch 1
mit:
(i) den Nucleotidsequenzen, gezeigt in SEQ-ID-No.1, SEQ-ID-No.4,
(ii) mindestens einer dieser Sequenzen, die den jeweiligen Sequenzen (i) innerhalb des Bereichs der Degeneration des genetischen Codes entsprechen oder
(iii) mindestens einer dieser Sequenzen, die mit den zu jeweiligen Sequenzen (i) oder (ii) komplementären Sequenzen hybridisieren und gegebenenfals.
(iiii) funktionsneutralen Sinnmutationen in i).

3. Mikroorganismen, insbesondere der Gattung Corynbacterium, transformiert durch die Einführung einer oder mehrerer der replizierbaren DNA gemäß einem der Ansprüche 1 oder 2.

4. Pendelvektor (shuttle vector) pECM3ilvBNCD,
**gekennzeichnet**
durch die in der Abb. 3 wiedergegebene Restriktionskarte, hinterlegt als E.coli DH5αmcr/pECM3ilvBNCD unter der Bezeichnung DSM 12457.

5. Pendelvektor (shuttle vector) pEKEx2panBC,
**gekennzeichnet**
durch die in der Abb.2 wiedergegebene Restriktionskarte, hinterlegt als E.coli DH5αmcr/pEKEx2panBC unter der Bezeichnung DSM 12456.

6. Verfahren zur Herstellung von Pantothensäure,
**dadurch gekennzeichnet**,
daß man in den Mikroorganismen der Gattung Corynebacterium das panB- und panC-Gen und gegebenenfalls weitere für die entsprechenden Enzyme codierenden Nucleotidsequenzen verstärkt (überexprimiert) und diese Mikroorganismen zur Fermentation einsetzt.

7. Verfahren zur Hestellung gemäß Anspruch 6,
**dadurch gekennzeichnet**,
daß man zusätzlich das ilvD-Gen verstärkt (überexprimiert).

8. Verfahren gemäß den Ansprüchen 6 oder 7,
**dadurch gekennzeichnet**,
daß man zusätzlich die Gene ilvBNCD verstärkt (überexprimiert).

9. Verfahren gemäß den Ansprüchen 6 oder 7,
**dadurch gekennzeichnet**,
daß man zur Erzielung der Verstärkung die Kopienzahl der Gene bzw. Nucleotidsequenzen in den Mikroorganismen durch Transformation mit diesen Gene bzw. Nucleotidsequenzen tragenden Plasmidvektoren erhöht.

10. Verfahren gemäß den Ansprüchen 6 oder 7,
**dadurch gekennzeichnet**,
daß man zur Erzielung der Verstärkung die sich stromaufwärts des Strukturgens befindende Promoter- und Regulationsregion mutiert.

11. Verfahren gemäß den Ansprüchen 6 oder 7,
**dadurch gekennzeichnet**,
daß man zur Erzielung der Verstärkung stromaufwärts des Strukturgens Expressionskassetten einbaut.

12. Verfahren gemäß den Ansprüchen 6 oder 7,
**dadurch gekennzeichnet**,
daß man zur Erzielung der Verstärkung die Lebensdauer der mRNA, die von den oben genannten Sequenzen als Matrize abgelesen wird, verlängert und/oder den Abbau der (des) entsprechenden Enzymproteins(e) verhindert.

13. Verfahren gemäß den Ansprüchen 6 bis 12,
**dadurch gekennzeichnet**,
daß man die Gene gemäß Anspruch 1 in Corynebacterien überexprimiert, die weitere Metabolit- bzw. Antimetabolit-Resistenzmutationen aufweisen.

14. Verfahren gemäß den Ansprüchen 6 bis 12,
**dadurch gekennzeichnet**,
daß man zur Erzielung der Überexpression das Kulturmedium und/oder die Fermentationsführung ändert.

15. Verfahren gemäß den Ansprüche 6 bis 14,
**dadurch gekennzeichnet**,
daß man zumindest einen der Stoffwechselwege in den Mikroorganismen ausgeschaltet, die die Pantothenat-(Pantothensäure)-bildung verringern.

16. Verfahren gemäß den Ansprüchen 6 bis 15,
**dadurch gekennzeichnet**,
daß man Mikroorganismen einsetzt, in denen man zusätzlich zu einem oder mehren der Gene die übrigen Gene des Stoffwechselweges der Pantothensäurebildung, einzeln oder gemeinsam, überexprimiert.

17. Verfahren gemäß Anspruch 15,
**dadurch gekennzeichnet**,
daß man im Stoffwechselweg das ilvA-Gen ausschaltet.

18. Verfahren gemäß einem oder mehreren der Ansprüche 6 bis 17,
**dadurch gekennzeichnet**,
daß man Mikroorganismen der Gattung Corynebacterium einsetzt, die den Pendelvektor pECM3ilvBNCD enthalten.

19. Verfahren gemäß einem oder mehreren der Ansprüche 6 bis 17,
**dadurch gekennzeichnet**,
daß man Mikroorganismen der Gattung Corynebacterium einsetzt, die den Pendelvektor pEKEx2panBC enthalten.

20. Verfahren zur Herstellung von Pantothensäure durch Fermentation von Mikroorganismen der Gattung Corynebacterium gemäß einem oder mehreren der vorhergehenden Ansprüche,
**dadurch gekennzeichnet**,
daß man
a) zumindest eines der Gene panB oder panC, bevorzugt panBC, gegebenfalls in Kombination mit dem ilvD-Gen, verstärkt (überexprimiert), und
b) die Pantothensäure im Medium oder in den Zellen der Mikroorganismen anreichert, und
c) die Pantothensäure isoliert.

21. Verfahren gemäß den Ansprüchen 19 und 20,
**dadurch gekennzeichnet**,
daß man während der Fermentation eine Vorstufe der Pantothensäure zusetzt, ausgewählt aus der Gruppe Aspartat, β-Alanin, Ketoisovalerat, Ketopantoat oder Pantoat.
